# EUROPEAN PATENT APPLICATION

(11) **EP 0 721 784 A1**
(43) Date of publication of application: **17.07.1996**
(21) Application number: 95926514.1
(22) Date of filing: 28.07.1995
(51) Int. Cl.: A61K 47/34

(54) **CONTROLLED-RELEASE DRUG PREPARATION**

(30) Priority: 29.07.1994 JP 178979/94; 29.07.1994 JP 179017/94; 08.09.1994 JP 214543/94
(71) Applicant: KANEGAFUCHI KAGAKU KOGYO KABUSHIKI KAISHA, Kita-ku Osaka-shi Osaka 530 (JP)
(72) Inventor: SAKAMOTO, Yoshitomo, Akashi-shi Hyogo 673 (JP); IMAI, Naohiro, Takasago-shi Hyogo 676 (JP); MASUDA, Shigeki, Akashi-shi Hyogo 674 (JP); NISHIMOTO, Takehiro, Osaka-shi Osaka 533 (JP); TANI, Nobutaka, Osaka-shi Osaka 545 (JP)
(74) Representative: VOSSIUS & PARTNER
(86) International application number: JP9501502
(87) International publication number: WO9604014

(57) **Abstract**

A controlled-release drug preparation comprising a composition containing an active ingredient (in particular, a remedy for inflammatory intestinal diseases) and coated with a copolymer composed of at least one type of structural unit represented by general formula (I) (wherein R represents a divalent organic group, and R¹ represents a sugar residue decomposed or utilized in the large intestine) and at least one type of structural unit represented by general formula (II) (wherein R represents a divalent organic group, X¹ and X² represent each independently oxygen, imino or sulfur, and R² represents a saturated or unsaturated hydrocarbon group, polyalkylene, polyalkylene glycol, polyarylene oxide, polyester or polyamide group); a controlled-release drug preparation; and a coating composition used therefor. The drug does not undergo, even when orally administered, decomposition and absorption anywhere other than in the large intestine and can reach the large intestine selectively without fail. Therefore, the preparation is effective in reducing the dose and side effects of the drug and improving bioavailability.

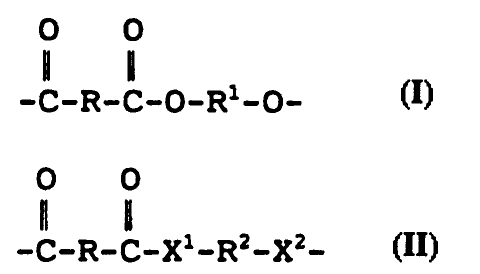

## Description

### TECHNICAL FIELD

This invention relates to a drug release-controlling preparation for releasing in the large intestine an active component having a pharmacological action after its oral administration and to a coating composition for making said drug release-controlling preparation. More particularly, it relates to a drug release-controlling preparation for oral administration in which an active component, especially a drug to be used for the treatment of inflammatory intestinal diseases, is coated with a copolymer which is degraded or assimilated by saccharide-hydrolyzing enzymes etc. in the large intestine.

### BACKGROUND ART

Though protein·peptide drugs such as insulin, vasopressin and the like are currently administered by intravenous injection, more simple and easy administration methods are demanded as these protein·peptide drugs have come into wide use, so that oral, transdermal, transnasal, suppository preparations and the like are being examined. Particularly, oral administration has been widely examined as the most general method. However, it has a disadvantage of having an extremely low bioavailability, because orally administered protein·peptide drugs are easily hydrolyzed and inactivated by enzymes in the small intestines.

Therefore, attempts have been made to improve the bioavailability by making these protein·peptide drugs into such preparations that they are released selectively in the large intestine where digestive enzymes are hardly present.

As a means to improve the bioavailability of protein·peptide drugs, there are enteric coated preparations which are designed in such a manner that active components in the preparations are released in the large intestine caused by dissolution of the coat film due to high pH in the organ, but such preparations have a problem in that they are disintegrated in the upper part of the small intestines or, on the contrary, excreted without disintegration, because the living body has a large daily pH fluctuation in the digestive organ and influences of food is large.

As another large intestine-selective releasing preparation, studies have been carried out on the preparations using polymers which are specifically degraded in the large intestine by enzymes secreted by bacteria in the large intestine. Various types of bacteria are present in the large intestine and secrete various enzymes which are different from human digestive enzymes. As an example of such enzymes, there is an enzyme which reduces azo groups into amino groups, and a polymer which contains in its molecule aromatic azo groups that are cut by this azo reducing enzyme has been reported (U.S. Patent No. 663,308; an unexamined published Japanese patent application (*Kokai*) No. 3-7718 corresponding to a published European patent application EP-A-398472). These methods, however, have disadvantages in that the polymer to be used has poor coat film-forming ability or solvent solubility and that azo groups having carcinogenicity are used.

Also, it is known that polyesters obtained from certain saccharides specifically degdarable in the large intestine and dibasic acids can show a large intestine-specific degradation property, but these polymers can be dissolved only in non-volatile aprotic polar solvents such as dimethylsulfoxide (DMSO), N,N-dimethylformamide (DMF), N,N-dimethylacetamide (DMAc) and the like and can merely be suspend in the volatile solvents such as acetone, ethanol, dichloromethane and the like which are useful for the coating of drugs, thus having problems in that complex handling is required at the time of the coating of drugs and uniform coat film cannot be formed easily.

In addition, it is known that the thickness of the coat film influence the releasing characteristics of the preparations or oral administration whose active components are released selectively in the large intestine. That is, when the coat film is too thick, these preparations are excreted as such prior to the release of active components by degradation of the coat film, and, when the film is too thin, they are disintegrated to cause release of the active components before reaching the large intestine due to mechanical stress and the like. However, when the aforementioned high polymer substance is used in the coat film, concentration of the high polymer substance dissolved in a solvent is small due to limitation in the kinds of solvent and the thus obtained solution is highly viscous, so that the resulting coating composition has poor fluidity which entails generation of irregularity at the time of the film formation and causes difficulty in exactly controlling the film thickness.

Typical examples of inflammatory intestinal diseases include ulcerative colitis and Crohn disease. Ulcerative colitis is a disease which shows a change to a diffuse inflammatory lesion on the mucosa membrane or submucosal layer of the large intestine. Crohn disease is a disease which shows a change to a granulomatous inflammatory lesion with fibrosis or ulcer on various sites of digestive tracts, with frequent cases of local inflammation on the large intestine.

These diseases have been treated, for example, by oral administration, intravenous injection or intestinal administration of a steroid drug such as prednisolone or the like or by oral or rectal administration of salazopyrin. It has been indicated that each of these drugs shows markedly high therapeutic effects when topically administered to the inflammation sites by rectal or intestinal administration.

Steroid agents including prednisolone are considerably important for the drug therapy of inflammatory intestinal diseases, because they are effective for the treatment of not only serious inflammatory intestinal diseases but also slight or moderate salazopyrin-resistant inflammatory diseases. However, it is necessary to pay careful attention when these drugs are used by oral administration or intravenous injection because of their high frequency to cause side effects. On the other hand, it has been shown that their therapeutic effects are markedly high when they are topically administered to the inflammation site or neighboring area thereof by rectal or intestinal administration. Such types of administration, however, still have problems remaining unsolved, such as limited applicable cases and complex administration method.

In the case of salazopyrin, it has been revealed that its active body is 5-aminosalicylic acid (to be referred to as "5-ASA" hereinafter), and therapeutic preparations containing 5-ASA as the active ingredient have been developed and used for the treatment of ulcerative colitis and Crohn disease. However, it is difficult to obtain sufficient therapeutic effects when 5-ASA is administered by general form of oral administration, because 5-ASA is degraded or absorbed in the upper regions of digestive tracts.

Therefore, a pharmaceutical designing has been made to prevent release and degradation of active ingredients in the upper parts of digestive tracts by coating an active ingredient-containing composition with an enteric or water insoluble coating material. Such preparations, however, are still insufficient in terms of selective drug release and effective release of active ingredients in the large intestine.

In consequence, great concern has been directed toward the development of a pharmaceutical preparation which has low side effects and high therapeutic effects, and can be easily administered, which renders possible effective topical administration of these steroid drugs and salazopyrin to the site of intestinal diseases or its neighboring area through their oral administration.

With the aim of finding a pharmaceutical preparation which is excellent in safety and can release active ingredients selectively in the large intestine, the inventors of the present invention have conducted extensive studies and found that drug release-controlling preparation suitable for the object can be produced by uniformly coating a drug-containing composition with a coat film having a proper thickness, using a coating composition containing a copolymer which is excellent in film-forming property, can be specifically degraded or assimilated in the large intestine and is excellent in safety; or by making a drug and said copolymer into a mixture (preferably a film).

Furthermore, with the aim of finding a coating composition which makes it possible to prepare a pharmaceutical preparation and to form of an uniform coat film by a simple procedure using said copolymer, the present inventors have continued extensive studies and, as the result, succeeded in accomplishing the coating composition of the present invention by dissolving or dispersing the aforementioned copolymer in a specific types of solvents with a specific mixing ratio.

In the treatment of diseases which show a change to an inflammatory lesion in the large intestine (e.g., ulcerative colitis and Crohn disease), it is expected that therapeutic effects could be increased and side effects could be reduced if the therapeutic drugs such as salazopyrin, 5-ASA, prednisolone and the like could be delivered selectively to the large intestine by oral administration. In consequence, the present inventors have conducted extensive studies with the aim of creating such an oral administration preparation effective for the treatment of inflammatory intestinal diseases such as ulcerative colitis and, as the result, have accomplished a preparation for oral administration which is physically and chemically stable and substantially can keep a drug for the treatment of inflammatory intestinal diseases inside the preparation while passing through upper digestive tracts (i.e., the stomach and the small intestines) and can release the drug accurately and efficiently after reaching the large intestine, by coating a composition containing an inflammatory intestinal disease treating drug with a coat film having a specific thickness, using a copolymer whose main chain is composed of a saccharide which is degraded or assimilated in the large intestine and certain compounds (an unexamined published Japanese patent application (*kokai*) No. 5-148348 corresponding to EP-A-542299) or using a composition containing said copolymer.

### DISCLOSURE OF THE INVENTION

Accordingly, the present invention provides a drug release-controlling preparation which comprises a copolymer comprising at least one structural unit represented by the general formula (I): 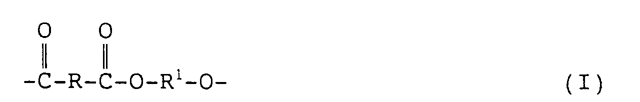
(wherein R represents a divalent organic group and R¹ represents a saccharide residue which is degraded or assimilated in the large intestine) and at least one structural unit represented by the general formula (II): 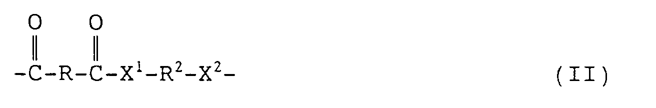
(wherein R represents a divalent organic group, X¹ and X² independently represent an oxygen atom, an imino group or a sulfur atom, and R² represents a saturated or unsaturated hydrocarbon group, a polyalkylene group, a polyalkylene glycol group, a polyarylene oxide group, a polyester group or a polyamide group), and a composition which contains an active ingredient, wherein said composition is coated with said copolymer.

The present invention also provides a drug release-controlling preparation which comprises a mixture of an active ingredient and said copolymer.

The present invention also provides a coating composition which comprises a solvent and a copolymer comprising at least one structural unit represented by the aforementioned general formula (I) and at least one structural unit represented by the aforementioned general formula (II), said copolymer being dissolved and/or dispersed in said solvent.

The present invention also provides a large intestine-selective and efficient preparation for oral administration which is used in the treatment of inflammatory intestinal diseases, which comprises a polymer that is degraded selectively in the large intestine, particularly a copolymer comprising at least one structural unit represented by the aforementioned general formula (I) and at least one structural unit represented by the aforementioned general formula (II), and a composition containing a drug for the treatment of inflammatory intestinal diseases, wherein said composition is coated with said copolymer.

The present invention also provides:
(1) the aforementioned drug release-controlling preparation and coating composition, wherein the aforementioned R¹ is an oligosaccharide residue which comprises at least two saccharide residues that are degraded or assimilated in the large intestine,
(2) the aforementioned drug release-controlling preparation and coating composition, wherein the aforementioned R¹ is a saccharide residue which is hydrolyzed by β-glucosidase, β-galactosidase or β-glucuronidase,
(3) the aforementioned drug release-controlling preparation and coating composition, wherein the aforementioned R¹ is a galacto-oligosaccharide residue, a fructo-oligosaccharide residue or a soybean oligosaccharide residue,
(4) the aforementioned drug release-controlling preparation and coating composition, wherein the aforementioned R¹ is a cellobiose residue,
(5) the aforementioned drug release-controlling preparation and coating composition, wherein the aforementioned structural unit (I) of the polymer is a structural unit obtained by ester-bonding a dibasic acid represented by HOOC-R-COOH and a saccharide represented by HO-R¹-OH, and the aforementioned structural unit (II) of the polymer is a structural unit obtained by ester-bonding a dibasic acid represented by HOOC-R-COOH and a polyalkylene glycol represented by HO-R²-OH,
(6) the aforementioned drug release-controlling preparation and coating composition, wherein the dibasic acid represented by HOOC-R-COOH constituting the aforementioned structural unit (I) of the polymer is terephthalic acid or isophthalic acid and the saccharide represented by HO-R¹-OH constituting the same is cellobiose, and the dibasic acid represented by HOOC-R-COOH constituting the aforementioned structural unit (II) of the polymer is terephthalic acid or isophthalic acid and the polyalkylene glycol represented by HO-R²-OH constituting the same is polytetramethylene glycol or polypropylene glycol,
(7) the aforementioned drug release-controlling preparation, wherein the coat film of the aforementioned copolymer has a thickness of approximately from 5 to 300 µm,
(8) the aforementioned drug release-controlling preparation, wherein its dosage form is tablets, capsules or granules,
(9) the aforementioned drug release-controlling preparation, wherein the aforementioned drug for the treatment of inflammatory intestinal diseases is a steroid drug or a pharmaceutically acceptable salt thereof,
(10) the aforementioned drug release-controlling preparation, wherein the aforementioned drug for the treatment of inflammatory intestinal diseases is prednisolone or a pharmaceutically acceptable salt thereof,
(11) the aforementioned drug release-controlling preparation, wherein the aforementioned drug for the treatment of inflammatory intestinal diseases is aminosalicylic acid or an aminosalicylic acid derivative, or a pharmaceutically acceptable salt thereof,
(12) the aforementioned coating composition, wherein the aforementioned copolymer is dissolved and/or dispersed in a solvent in a concentration of from 0.1 to 50% by weight based on the total weight of the coating composition, and
(13) the aforementioned coating composition, wherein said solvent comprises at least one solvent selected from the group consisting of water, alcohols having 1 to 5 carbon atoms, aromatic hydrocarbons, esters, ketones, ethers, halides and glycols.

The present invention provides a drug release-controlling preparation in which the aforementioned copolymer to be used as its coat film contains a saccharide which is degraded or assimilated specifically in the large intestine, so that it is resistant against chemical and enzymatic degradation in the stomach and small intestines and can release an active ingredient (e.g., a drug or other biologically active substance) in the large intestine selectively and accurately.

The present invention is a drug release-controlling preparation which is obtained by coating a composition containing an active ingredient (e.g., a drug or other biologically active substance) with a coating composition containing the aforementioned copolymer in a proper film thickness. The proper film thickness is selected in accordance with the shape and size of the composition which contains an active ingredient and the like and the intended site to which the active ingredient is delivered.

Also, the present invention is a drug release-controlling preparation which is characterized in that thickness of the coat film comprising the aforementioned copolymer is 5 µm or more and 300 µm or less.

If the coat film is too thin with respect to the pharmaceutical preparation of the present invention coated with a base material containing a copolymer which is specifically degraded or assimilated by a saccharide-hydrolyzing enzyme, it is disintegrated and its active ingredient is released before the preparation reaches the large intestine due to friction and the like caused by the movement in the digestive tracts and its contact with contents in the tracts. For example, when the coat film thickness is less than 5 µm, most portion of the preparation is disintegrated in the upper digestive tracts to release its active ingredient. When the film thickness is 5 µm or more and less than 20 µm, disintegration of several % to a little over 10% of the administered preparation is observed in the upper digestive tracts and, when leaked active ingredient is taken into consideration, the part which is not biologically utilized reaches 10 to 20%. However, disintegration of the preparation and leakage of its active ingredient do not occur in the upper digestive tracts when coated with a film thickness of 20 µm or more. In addition, the period of time until the preparation coated with the aforementioned copolymer-containing base material is disintegrated after reaching the large intestine is dependent upon the film thickness, and the preparation is excreted before it is disintegrated to release the active ingredient when the coat film is too thick. In fact, all preparations were disintegrated and its active ingredient was released in the large intestine when the film thickness was 20 µm or more and 150 µm or less. However, excretion of a small number of preparations without disintegration is observed when the thickness is more than 150 µm and equal to or lower than 300 µm, and most preparations are excreted without disintegration and the active ingredient are not released when the film thickness exceeds 300 µm.

In consequence, thickness of the coat film suitable for effecting selective and accurate release of an active ingredient such as a drug or other biologically active substance in the large intestine is preferably 5 µm or more and equal to or lower than 300 µm, more preferably 10 µm or more and equal to or lower than 200 µm, most preferably 20 µm or more and equal to or lower than 150 µm.

The present invention also provides a coating composition used in the delivery system of an active ingredient or other biologically active substance to the large intestine, which comprises a copolymer comprising a saccharide which is degraded or assimilated in the large intestine and having resistance against chemical and enzymatic degradation in the stomach and small intestines is dissolved or dispersed in a solvent.

Since the copolymer used in the present invention is excellent in solvent solubility and (or) dispersing ability, handling at the time of the production of a pharmaceutical preparation can be simple and easy, and an uniform coat film can be formed stably and the production loss is extremely small. In addition, compositions having different volatility and viscosity at the same concentration can be produced by the use of a mixture system of various solvents, and the dissolution concentration and dispersion concentration can be controlled at will by changing the kinds and mixing ratio of the solvents. In consequence, the coating composition of the present invention can be used as a coating material for the production of large intestine-selective release preparations and is characterized in that the aforementioned copolymer is dissolved or dispersed in a solvent in an amount of from 0.1 to 50% (% by weight; unless otherwise noted, % as used in this specification means % by weight) based on the total weight of the coating composition.

Next, the copolymer to be used in the present invention is described.

The copolymer to be used in the present invention is a copolymer which comprises at least one structural unit represented by the general formula (I): 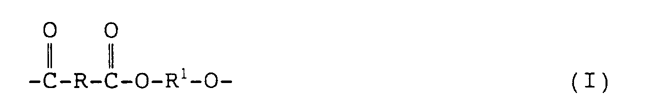
(wherein R represents a divalent organic group and R¹ represents a saccharide residue which is degraded or assimilated in the large intestine) and at least one structural unit represented by the general formula (II): 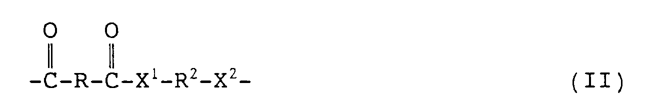
(wherein R represents a divalent organic group, X¹ and X² independently represent an oxygen atom, an imino group or a sulfur atom, and R² represents a saturated or unsaturated hydrocarbon group, a polyalkylene group, a polyalkylene glycol group, a polyarylene oxide group, a polyester group or a polyamide group).

The copolymer used in the present invention is not particularly limited to a straight chain copolymer and may have a partially branched or cross-linked structure, with the proviso that required dosage forms can be produced. It is possible to produce a copolymer mainly composed of a straight chain structure having a low degree of branching or cross-linking, making use of the difference in reactivities of primary and secondary alcohols in oligomers during the production steps. In that case, when a gelated substance (cross-linked body having a network structure) is partially formed, it can be removed by dissolving the product in a solvent and filtering off the insoluble matter. In addition, the copolymer may be either a random copolymer or a block copolymer.

Though not particularly limited, molecular weight of the copolymer may be in the range of preferably from 2,000 to 1,000,000, more preferably from 5,000 to 500,000, most preferably from 10,000 to 300,000.

Unless otherwise noted, the term "molecular weight" as used herein means weight average molecular weight which, for example, can be measured as styrene-based weight average molecular weight by known methods.

Copolymerization ratio of the two structural units (I) and (II) as their molar ratio may be within the range of preferably from 1:99 to 99:1, more preferably from 5:95 to 80:20, most preferably from 10:90 to 70:30. Binding direction of the structural units (I) and (II) is not particularly limited.

Preferably, the copolymer of the present invention comprises at least 2 units of the structural unit represented by the formula (I) and at least 1 unit of the structural unit represented by the formula (II).

The structural unit represented by the general formula (I) is obtained by allowing a dibasic acid represented by HOOC-R-COOH to form ester bonds with a saccharide represented by HO-R¹-OH.

A large variety of bacteria are present in the large intestine, and recent studies have revealed that these bacteria can ferment and utilize hardly digestible saccharides which cannot or can hardly be digested and absorbed in the human body, so that such saccharides previously thought to be unusable in the human body are absorbed through this process. In addition, the presence of specific saccharide-hydrolyzing enzymes in the large intestine has been drawing attention through studies on the formation of carcinogenic substances in the human body. Examples of such hydrolases include saccharide hydrolyzing enzymes such as β-glucosidase, β-galactosidase and β-glucuronidase, as well as xylanase, β-glucanase, galactomannanase, polygalacturonase, mucinase, chondroitin lyase, carboxymethylcellulase, cellulase, polygalacturonate lyase and the like.

The saccharide represented by HO-R¹-OH is a hardly digestible saccharide composed of 2 or more and preferably 100 or less, more preferably 50 or less, most preferably 10 or less of saccharide residues, which is degraded or assimilated in the large intestine. R¹ represents a saccharide residue of such saccharide. The term "hardly digestible saccharide" as used herein means a saccharide which is hardly digested and absorbed or not digested in the human small intestines.

Examples of such saccharide include those which are hydrolyzed by the aforementioned enzymes. The illustrative examples thereof include, though not particularly limited, galacto-oligosaccharides in which several molecules of galactose are linked to the galactose residue of lactose by β-1,4 or β-1,6 bonding (e.g., 6'-galactosyl lactose, 4'-galactosyl lactose, and the like); fructo-oligosaccharides in which several molecules of fructose are linked to the fructose residue of sucrose by α-1 or β-2 bonding (e.g., 1-kestose, nistose, and the like); soybean oligosaccharides in which several molecules of galactose are linked to the glucose residue of sucrose by β-1,6 bonding (e.g., raffinose, stachyose, and the like); xylo-oligosaccharides in which several molecules of xylose are linked by β-1,4 bonding (e.g., xylobiose, xylotriose, and the like); isomalto-oligosaccharides in which several molecules of glucose are linked to the non-reducing glucose of isomaltose by α-1,4 or α-1,6 bonding (e.g., isomaltose, isomaltotriose, panose, and the like); oligocellulose in which several molecules of glucose are linked by β-1,4 bonding (e.g., cellobiose, cellotriose and the like); disaccharide alcohols such as lactose, lactosucrose, lacturose, palatinose, melezitose, turanose, melibiose, maltitol, lactitol, isomaltitol, glucopyranosyl-α-1,6-mannitol and the like; and carboxymethylcellulose, guar gum, tragacanth gum, xylan and the like.

Among these saccharides, saccharides, galacto-oligosaccharides, fructo-oligosaccharides or soybean oligosaccharides which are hydrolyzed by β-glucosidase, β-galactosidase or β-glucuronidase are preferred. Especially, saccharides composed of 5 or less saccharide residues such as cellobiose, lactose, raffinose, stachyose and the like are preferred, more preferably disaccharides, most preferably cellobiose. These saccharides are available as commercial products.

These saccharides may be modified with an acyl group (RCO- wherein R is a hydrocarbon group) such as acetyl, propionyl, stearoyl, phthaloyl, adipoyl, malonyl, benzoyl or the like; an alkyl group such as methyl, ethyl or the like group; or a hydroxyalkyl group such as hydroxyethyl, hydroxypropyl or the like. The number of molecules of these modification groups to be linked to the saccharide is not particularly limited, provided that at least two un-modified hydroxyl groups remain in one molecule of the saccharide, and the saccharide may be modified with one or more kinds of modification groups.

The copolymer of the present invention may contain two or more kinds of the aforementioned saccharide HO-R¹-OH, but preferably one kind.

In the dibasic acid represented by HOOC-R-COOH, R represents a divalent organic group or a single bond, and examples of such an organic group include aromatic hydrocarbons and derivatives thereof (preferably those having 6 to 30 carbon atoms), aliphatic hydrocarbons and derivatives thereof (preferably those having 1 to 30 carbon atoms) and alicyclic hydrocarbons and derivatives thereof (preferably those having 3 to 30 carbon atoms). Illustrative examples of the aromatic hydrocarbon include a benzene ring, a naphthalene ring, 1,2-bisphenoxyethane and the like, those of the aliphatic hydrocarbon include methylene, ethylene, propylene, butylene and the like and those of the alicyclic hydrocarbon include cyclohexane and the like. Though not particularly limited, illustrative examples of the dibasic acid include preferably aromatic dibasic acids such as terephthalic acid, isophthalic acid, 4,4'-dicarboxybiphenyl, 2,6-dicarboxynaphthalene, 1,2-bis(phenoxy)ethane-4,4'-dicarboxylic acid, 1,2-bis(2-chlorophenoxy)ethane-4,4'-dicarboxylic acid and the like; aliphatic dibasic acids such as adipic acid, 1,10-decanedicarboxylic acid, diethylmalonic acid, fumaric acid, maleic acid, oxalic acid and the like; and alicyclic dibasic acids such as cyclohexane diacid and the like, more preferably terephthalic acid, isophthalic acid, adipic acid and oxalic acid, most preferably terephthalic acid and isophthalic acid.

The copolymer of the present invention may contain two or more of these dibasic acids, and R in the structural unit represented by the general formula (I) and R in the structural unit represented by the general formula (II) may be the same or different from each other, but preferable the same.

On the other hand, the structural unit represented by the general formula (II) is obtained by bonding the aforementioned dibasic acid represented by HOOC-R-COOH to a compound represented by H-X¹-R²-X²-H.

R² represents an unsaturated hydrocarbon group, a saturated hydrocarbon group (except for polyalkylene), a polyalkylene group, a polyalkylene glycol, a polyarylene oxide group, a polyester group or a polyamide group.

The following illustratively describes the compound H-X¹-R²-X²-H by dividing it into four types (general formulae (III) to (VIII)).
(1) A compound represented by the general formula (III)

   H-X¹-R³-X²-H (III)

   wherein X¹ and X² independently represent an oxygen atom, an imino group or a sulfur atom, R³ represents a saturated hydrocarbon group (preferably having 1 to 30 carbon atoms) or an unsaturated hydrocarbon group (preferably having 2 to 30 carbon atoms). That is, a saturated or unsaturated hydrocarbon compound having a hydroxyl group, an amino group or a thiol group independently on its both ends. Its illustrative examples include alkylene glycols such as ethylene glycol, propylene glycol, 1,2-butanediol, 1,3-butanediol, 1,4-butanediol and the like; alkylene diamines such as hexamethylenediamine, ethylenediamine, tetramethylenediamine and the like; alkane dithiols such as 1,4-butanedithiol, ethanedithiol and the like; aromatic diols such as 1,4-hydroquinone, 1,3-hydroquinone, 2,7-dihydroxynaphthalene, catechol, 1,3-di(hydroxymethyl)benzene and the like; and aromatic diamines such as 1,4-diaminobenzene, 3,5-diaminotoluene and the like.
(2) A compound represented by the general formula (IV):

   H-X¹-(R⁴O)ₙ-R⁵-X²-H (IV)

   wherein X¹ and X² are as defined above, R⁴ and R⁵ independently represent an alkylene group (preferably those having 1 to 10 carbon atoms) or an aryl group (preferably those having 6 to 20 carbon atoms) and n is an integer of 1 to 100. That is, a polyalkylene glycol or a polyarylene oxide having a hydroxyl group, an amino group or a thiol group on their both ends. Their illustrative examples include derivatives of polyethylene glycol, polypropylene glycol, polytetramethylene glycol, polyphenylene oxide and the like, each having a hydroxyl group, an amino group or a thiol group on its both ends.
(3) A compound represented by the general formula (V) or (VI): 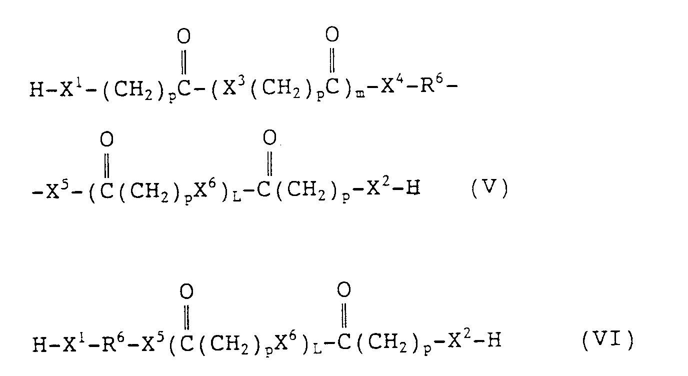 (wherein X¹ and X² are as defined above, X³ and X⁶ independently represent an oxygen atom or an imino group, X⁴ and X⁵ independently represent an oxygen atom, a sulfur atom or an imino group, R⁶ represent an alkylene group (preferably those having 1 to 20 carbon atoms), a divalent group of an aromatic compound (preferably those having 2 to 20 carbon atoms) or an alkylene glycol group (preferably those having 6 to 20 carbon atoms), m and L are independently an integer of 0 to 100 and p is an integer of 1 to 12, and R⁶ and R⁵ in the formula (V) are linked by a single bond), or a compound represented by the general formula (VII): 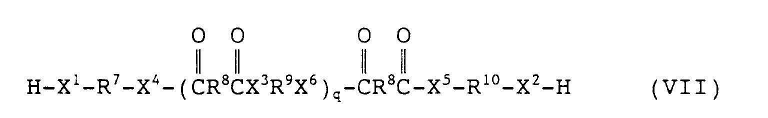 (wherein X¹, X², X³, X⁴, X⁵ and X⁶ are as defined above, R⁷, R⁹ and R¹⁰ are independently an alkylene group (preferably those having 1 to 20 carbon atoms) or a divalent group of an aromatic compound, R⁸ represents-a divalent organic group and q is an integer of 1 to 100). That is, when X³ and X⁶ are oxygen atoms, it is a polyester having a hydroxyl group, an amino group or a thiol group independently on its both ends, such as poly ε-caprolactone, polyglycolic acid, polylactic acid, polyethylene terephthalate, polybutylene terephthalate, polyethylene adipate and the like, each having a hydroxyl group, an amino group or a thiol group on its both ends and, when X³ and X⁶ are imino groups, it is a polyamide having a hydroxyl group, an amino group or a thiol group independently on its both ends, such as poly ε-caprolactam, hexamethylenediamine-adipic acid copolymer and the like, each having a hydroxyl group, an amino group or a thiol group on its both ends.
(4) A compound represented by the general formula (VIII):

   H-X¹-(CH₂CR¹¹R¹²)ᵣ-X²-H (VIII)

   (wherein X¹ and X² are as defined above, R¹¹ and R¹² are independently a hydrogen atom, an alkyl group, an acetyl group, a phenyl group, a phenyl group substituted with 1 to 4 alkyl groups or nitro groups, a naphthalene group, a carboxyl group, a carboxymethyl group, a carboxyethyl group, a carboxyisopropyl group, a carboxy-2-hydroxypropyl group, a methoxycarbonyl group, an ethoxycarbonyl group, an isopropoxycarbonyl group, a 1-hydroxyethoxycarbonyl group or a 2-hydroxypropoxycarbonyl group and r is an integer of 1 to 1,000. That is, a polyalkylene having a hydroxyl group, an amino group or a thiol group independently on its both ends. Its illustrative examples include polystyrene, polyvinyl acetate, methyl polymethacrylate, hydroxyethyl polymethacrylate and the like, each having a hydroxyl group, an amino group or a thiol group on its both ends.

These compounds can be prepare, for example, in the following manner. The compounds of the general formulae (III) and (IV) can be obtained, for example, as commercial products. The compound of general formula (V) can be prepared, for example, by ring-opening polymerization of caprolactone or the like using ethylene glycol or the like as an initiator. The compound of general formula (VI) can be prepared, for example, by ring-opening polymerization of caprolactone or the like using one side-protected ethylene glycol or the like as an initiator. The compound of general formula (VII) can be prepared, for example, by condensation of a dibasic acid with ethylene glycol, ethylenediamine or the like. The compound of general formula (VIII), such as polystyrene, polyvinyl acetate, methyl polymethacrylate or the like, can be obtained, for example, as a commercial product.

These compounds illustrated above are only examples and the compounds represented by H-X¹-R²-X²-H are not restricted thereby. A polyether having a hydroxyl group on its both ends, such as polypropylene glycol or polytetramethylene glycol, or a polyester having hydroxyl group on its both ends, such as ε-caprolactone or polyglycolic acid, is most desirable as a compound having improved solvent solubility and high safety. Preferably, the structural unit (I) of the aforementioned copolymer is a structural unit obtained by the ester bonding of a dibasic acid represented by HOOC-R-COOH and a saccharide represented by HO-R¹-OH, and the structural unit (II) is a structural unit obtained by the ester bonding of a dibasic acid represented by HOOC-R-COOH and a polyalkylene glycol represented by HO-R²-OH. More preferably, the dibasic acid represented by HOOC-R-COOH constituting the structural unit (I) of the aforementioned copolymer is terephthalic acid or isophthalic acid and the saccharide represented by HO-R¹-OH constituting the same is cellobiose, and the dibasic acid represented by HOOC-R-COOH constituting the structural unit (II) is terephthalic acid or isophthalic acid and the polyalkylene glycol represented by HO-R²-OH constituting the same is polytetramethylene glycol or polypropylene glycol.

Synthesis of the copolymer used in the present invention having such structural units is carried out in accordance with a known method (K. Kurita *et al*., *J*. *Polymer Chem*. *Ed*., 18, 365, (1980)). That is, a saccharide, a copolymerizable compound and chloride of a dibasic acid are allowed to react one another, preferably at about 17°C, in a non-volatile aprotic polar solvent such as dimethyl sulfoxide (DMSO), N,N-dimethylformamide (DMF), N,N-dimethylacetamide (DMAc) or the like, using a tertiary amine such as pyridine, triethylamine or the like as a proton removing agent.

Suitable solvent for use in the coating composition of the present invention can optionally be selected taking its desired concentration (0.1 to 50% (w/w, the same shall apply hereinafter)), volatility and film-forming property into consideration and, though not particularly limited, it may be at least one solvent selected from the group consisting of water, an alcohol having 1 to 5 carbon atoms, an aromatic hydrocarbon, an ester, a ketone, an ether, a halide, an olefin, an amide, an oxide and a glycol, preferably at least one solvent selected from the group consisting of water, an alcohol having 1 to 5 carbon atoms, an aromatic hydrocarbon, an ester, a ketone, an ether, a halide and a glycol. More preferably, it may be at least one solvent or a mixture of two or more solvents selected from the group consisting of ethylene glycol, dichloromethane, dichloroethane, chloroform, methanol, ethanol, 1-propanol, 2-propanol, 1-butanol, 2-butanol, tert-butyl alcohol, 1-pentanol, toluene, xylene, methyl acetate, ethyl acetate, propyl acetate, isopropyl acetate, butyl acetate, isobutyl acetate, pentyl acetate, isopentyl acetate, acetone, diisobutyl ketone, methyl ethyl ketone and tetrahydrofuran; and most preferably it is tetrahydrofuran; a mixed solvent of methanol, ethanol or 2-propanol with toluene; a mixed solvent of methanol, ethanol or 2-propanol with ethyl acetate; a mixed solvent of methanol, ethanol or 2-propanol with methyl ethyl ketone; a mixed solvent of dichloromethane or chloroform with methanol; a mixed solvent of dichloromethane or chloroform with ethanol; water; a solvent selected from alcohols having 1 to 5 carbon atoms; methyl ethyl ketone; and a mixed solvent of methanol, ethanol or 2-propanol with acetone.

Concentration of the copolymer in the coating composition of the present invention varies depending on the composition of the copolymer and (or) the composition of the solvent and is not particularly limited, but, when solubility or dispersion ability of the copolymer in the solvent and its workability as a coating agent are taken into consideration, its concentration may be within the range of preferably from 0.1 to 50% by weight, more preferably from 1 to 30% by weight, based on the total weight of the coating composition. When concentration of the copolymer is less than 0.1% by weight, it will cause problems in that a large amount of solvent is required, pin holes are apt to be formed in the resulting coat film due to the increased amount of solvent and the number of times of the cyclic operation increases until a desired coat film is obtained. When the concentration is larger than 50% by weight, fluidity of the coating composition becomes poor due to increased viscosity, thus entailing decreased operation ability and inferior uniformity of the coat film.

The composition of the present invention is obtained by adding the aforementioned copolymer to a solvent and stirring the mixture to effect dissolution or dispersion of the copolymer. If necessary, the dissolution time can be shortened by heating the mixture in an appropriate concentration or finely pulverizing the aforementioned copolymer.

In addition to the aforementioned copolymer, a drug-containing composition and the drug release-controlling preparation and coating composition of the present invention may be further blended with various pharmaceutically acceptable compounding agents conventionally used in this field, such as an active ingredient, an excipient, a binding agent, a lubricating agent, a coagulation-preventing agent, a disintegrating agent, a foaming agent, an absorption-enhancing agent, a stabilizing agent, an active ingredient-solubilizing aid, a plasticizer and, as occasion demands, a high polymer substance other than the aforementioned copolymer.

Examples of the excipient include saccharides such as sucrose, lactose, mannitol, glucose, starch, crystalline cellulose and the like and calcium phosphate, calcium sulfate, calcium lactate and the like. Examples of the binding agent include polyvinyl alcohol, polyacrylic acid, polymethacrylic acid, polyvinyl pyrrolidone, glucose, sucrose, lactose, maltose, sorbitol, mannitol, hydroxyethylcellulose, hydroxypropylmethylcellulose, hydroxypropylcellulose, macrogols, acacia, gelatin, agar, starch and the like. Examples of the lubricating agent include stearic acid, talc and the like. As the coagulation-preventing agent, lubricating agents described above (e.g., stearic acid, talc and the like) are used preferably, as well as soft silicic anhydride, aqueous silicon dioxide and the like. Examples of the disintegrating agent include crystalline cellulose, carboxymethylcellulose, carboxymethylcellulose salts, starch, hydroxypropylstarch, carboxymethylstarch sodium, tragacanth gum and the like. Examples of the foaming agent include sodium bicarbonate, tartaric acid and the like. Examples of the absorption enhancing agent include surface active agents such as higher alcohols, higher fatty acids, glycerol fatty acid ester and the like. Examples of the stabilizing agent include benzoic acid, sodium benzoate, ethyl paraoxybenzoate and the like. Examples of the active ingredient-solubilizing aid include organic acids such as fumaric acid, succinic acid, malic acid and the like. Examples of the plasticizer include polyethylene glycol, polypropylene glycol, polytetramethylene ether glycol, glyceric acid ester, sucrose ester, castor oil, triethyl citrate, triacetin and the like. Examples of the high polymer substance other than the aforementioned copolymer include ethylcellulose, methacrylic acid copolymers, polyethylene glycol, polypropylene glycol, shellac and the like.

The coating composition of the present invention is a composition in which the aforementioned copolymer is dissolved or dispersed in an appropriate solvent. Since the copolymer of the present invention is degraded or assimilated specifically in the large intestine, a pharmaceutical preparation prepared using the composition of the present invention can release its active ingredient selectively in the large intestine with preventing the active ingredient from its degradation by digestive enzymes, resulting in accumulation of the active ingredient in the large intestine in a high concentration and enhancement of its absorption from the large intestine, so that the inventive composition can be used as a coating agent for pharmaceutical preparation for improving the bioavailability of pharmaceutically active ingredients. The term "coating agent for pharmaceutical preparation" as used herein includes outer coating agents for solid preparations for oral administration (tablets, granules, pills, capsules and the like) and sheet preparations in which drugs are embedded.

When the coating composition of the present invention is used as an outer coating agent, a separately produced drug-containing composition is coated with the aforementioned coating composition by a pan coating or fluidized coating methods.

The coat film obtained by using the coating composition of the present invention can be prepared into desired thickness, and the film thickness necessary for its specific and accurate disintegration in the large intestine may be preferably from 5 to 300 µm, more preferably from 10 to 200 µm, most preferably from 20 to 150 µm.

When the composition of the present invention is used as a sheet preparation, films are prepared from the aforementioned coating composition using a film-forming apparatus in the usual way. For example, each of a base material containing the aforementioned copolymer, an active ingredient, a plasticizer, a stabilizing agent, an absorption enhancing agent and the like; or a base material containing the aforementioned copolymer and a drug-containing composition comprising an active ingredient, a plasticizer, a stabilizing agent, an absorption enhancing agent and the like are dissolved or dispersed in a 1:1 (weight ratio) mixed solution of ethyl acetate and ethanol or a 1:1 (weight ratio) mixed solution of dichloromethane and ethanol, which is injected into a film-forming apparatus to form a coat film, and the thus formed coat film is dried to obtain a film sheet. By finely pulverizing the thus obtained film sheet, a mixture of a base material containing the aforementioned copolymer, an active ingredient, a plasticizer, a stabilizing agent, an absorption enhancing agent and the like or a mixture of a base material containing the aforementioned copolymer and a drug-containing composition comprising an active ingredient, a plasticizer, a stabilizing agent, an absorption enhancing agent and the like is prepared and packed in capsules and the like. As occasion demands, not only the aforementioned copolymer but also one or more of other high polymer substances (e.g., ethyl cellulose, methacrylic acid copolymers and the like) and (or) a plasticizer (polyethylene glycol or the like) may be mixed.

As the dosage form of the drug release-controlling preparation of the present invention, coated preparations coated with a base material containing the aforementioned copolymer are included. Since the aforementioned copolymer is degraded or assimilated specifically in the large intestine, degradation of drugs by digestive enzymes can be prevented by releasing them selectively in the large intestine and their absorption from the large intestine can be accelerated by accumulating them in the large intestine in a high concentration, so that the copolymer can be applied to coating preparations with the aim of improving the bioavailability of such drugs. The term "coating preparations" as used herein include drug-containing compositions, preferably pharmaceutical preparations prepared by coating solid preparations for oral administration (e.g., tablets, granules, pills, capsules and the like) with the aforementioned coating composition, and sheet preparations in which drugs are embedded.

All active substances such as drugs and other biologically active substances can be used with no particular limitation as drugs to be made into pharmaceutical preparations in the present invention, provided that the object thereof is to target the large intestine. Examples of such active ingredients include large intestine ulcer-treating drugs such as prednisolone, 5-aminosalicylic acid and the like and derivatives thereof, carcinostatic agents such as bleomycin and the like which show significant effects on large intestine cancer, protein or peptide drugs such as insulin, vasopressin, calcitonin, human granulocyte colony-stimulating factor (G-CSF), erythropoietin, various types of interleukins and the like, guanidinobenzoic acid derivatives such as gabexate mesilate, camostat mesilate, nafamostat mesilate and the like and anti-inflammatory drugs such as aspirin and the like.

The present invention also relates to a drug release-controlling preparation which contains an inflammatory intestinal disease-treating agent as its active ingredient.

The inflammatory intestinal disease-treating agent according to the present invention is not particularly limited, provided that it exerts desired drug effect by its topical administration to the inflammation region or surrounding area thereof in the large intestine. Illustrative examples of such agents include steroid drugs such as prednisolone and the like, an ulcerative colitis-treating agent such as salazopyrin or 5-ASA which is considered to be its active body, derivatives thereof and not only their acid addition salts and hydrochlorides but also other organic acid salts and inorganic salts thereof, as well as their alkali metal salts with K, Na and the like and alkaline earth metal salts with Ca, Mg and the like which are formed by the carboxyl group in the molecule. Also included are non-steroidal anti-inflammatory drugs such as indomethacin and the like and similar anti-inflammatory drugs which have the activity to inhibit arachidonic acid metabolism, arachidonic acid metabolite antagonists such as LTB4 antagonist and the like, active oxygen-eliminating agents such as superoxide mutase and the like, active oxygen formation-inhibiting agents such as ascorbic acid and the like and cytokine antagonists such as interleukin 1 antagonist and the like. In addition, pharmaceutically acceptable salts of these drugs are also included.

The following describes the present invention further in detail with reference to prednisolone as the inflammatory intestinal disease-treating agent.

Prednisolone has been used widely as an anti-inflammatory disease-treating agent and also as an inflammatory intestinal disease-treating agent solely or in combination with an ulcerative colitis-treating agent, salazopyrin. Its administration form include oral administration, intravenous injection, rectal administration and intestinal administration. Its oral administration or intravenous injection can show therapeutic effects but causes serious problem of generating side effects due to the systemic administration. On the other hand, it is known that high therapeutic effects are obtained by its rectal administration. However, this route of administration is effective only when inflammation is found in the rectum, so that its application range is limited. It is known also that high therapeutic effects can be obtained by its intestinal administration, particularly in patients and serious cases having inflammation in the lower part of the large intestine, but it causes problems in that the administration method is complex, imposes a burden on patients and is not effective in the case of inflammation in relatively upper part of the large intestine.

In view of the above, a pharmaceutical preparation which can be administered topically to intestinal inflammation regions which similar to the rectal or intestinal administration but via its oral administration and also which does not cause generation of systemic side effects by the absorption of steroid components from the upper part of the digestive tracts, namely a preparation which makes it possible to selectively administer drugs into the large intestine by oral administration, becomes an effective agent for the treatment of inflammatory intestinal diseases, particularly ulcerative colitis and large intestine type Crohn disease. In order to realize this object, large intestine-selective pharmaceutical preparations produced making use of enteric base materials have been reported, but their effects are not sufficient.

In the present invention, an attempt was made to develop a new oral administration preparation by applying a base material which is degraded in the large intestine, as a means to realize selective delivery of prednisolone into the large intestine. As the application forms of a base material which is degraded in the large intestine, it may be used as a raw material of tablets, pills and the like or as a coating base material, but a release-controlling preparation in which a polymer that degrades in the large intestine is used as a coating material is more preferable when release of a drug after reaching the large intestine and its combination with other preparation techniques (for example, the use of a swelling material as a material of tablets) are taken into consideration.

In general, when a pharmaceutical preparation utilizing polymer degradable in the large intestine is coated with the polymer in a specific film thickness or more, it can be made into an oral administration preparation which is physically and chemically stable in the upper digestive tracts, namely the stomach and the small intestines, and can reach the large intestine while substantially keeping an inflammatory intestinal disease-treating drug inside the preparation.

However, in order to realize effective release of prednisolone as the active ingredient after reaching the large intestine, it is quite important to design the dosage form taking into consideration disintegration property of the coat layer and release of the active ingredient in the reached area, for example by adjusting thickness of the coated layer. The inventors of the present invention have created an oral solid preparation which is completely stable physically and chemically in the upper digestive tracts, can keep prednisolone substantially as such inside the preparation and is able to release prednisolone from the preparation accurately after reaching the large intestine and before excreting from the body, by coating a prednisolone-containing drug-containing composition with a polymer whose main chain is comprised of a saccharide which is degraded or assimilated in the large intestine and certain compounds or with a coat-forming composition containing said polymer, and by controlling the coat layer to a specific thickness. That is, it was found that it is preferable to control thickness of the coat film within the range of from 5 to 300 µm depending on the dosage form of each drug-containing composition (tablets, granules, pills, capsules or the like) which contains an inflammatory intestinal disease treating drug, though the proper thickness varies depending on each dosage form. When the coat film thickness is smaller than the above range, the preparation is disintegrated and its active ingredient is released before the preparation reaches the large intestine due to friction and the like caused by the movement in the digestive tracts and its contact with contents in the tracts. Also, when the thickness is larger than the above range, the preparation is excreted from the body before the active ingredient is released by degradation of the coat film.

In the case of the inflammatory intestinal disease treating agent, it is important that the drug released from the preparation directly act upon the inflammation tissue or its surrounding area from the viewpoint of exerting its proper therapeutic effects. In consequence, it is exceedingly important to design the dosage form in accordance with each inflammatory condition of the inflammatory intestinal disease to be treated. In other words, a preparation which releases prednisolone as soon as the preparation reaches the large intestine is preferable for the purpose of treating a disease in which the inflammation region is located in the relatively upper part of the large intestine, and a preparation which releases prednisolone after a certain period of time after the preparation reaches the large intestine is preferable for the purpose of treating a disease in which the inflammation region is located in the relatively lower part of the large intestine. When inflammation is distributed wholly in the large intestine, a preparation is desirable which releases prednisolone continuously after it is delivered to the large intestine until excreted from the body.

Such types of oral administration preparations are realized by more precisely controlling thickness of the aforementioned coat layer. That is, a film thickness of 5 to 100 µm is preferable for the purpose of treating an inflammatory intestinal disease in the relatively upper part of the large intestine, a film thickness of 50 to 300 µm is preferable for the purpose of treating an inflammatory intestinal disease in which the inflammatory region is located in the relatively lower part and a film thickness of 30 to 150 µm is preferable when the inflammation is distributed in the whole large intestine. In this connection, although the thickness in the above ranges causes no practical and functional problems, a coat layer having uniform thickness is preferable in these preparations having controlled coat layer thickness.

In addition, when the dosage form is granules for the treatment of an inflammatory intestinal disease in which the inflammation is distributed in the entire large intestine, the granules may be coated with films having different thicknesses. That is, an oral administration preparation having high therapeutic effects can be produced by packing capsules with a granule preparation prepared by mixing a preparation having a relatively thin coat layer thickness with a preparation having a relatively thick coat layer thickness or a mixture thereof.

### BEST MODE OF CARRYING OUT THE INVENTION

The following Reference Examples, Examples, Comparative Examples and Test Examples are provided to further illustrate the present invention, but not by way of limitation. In the following description, ratio, percentage and the like are by weight unless otherwise noted.

### Reference Example 1

342 mg (1.0 mmol) of cellobiose, 2.0 g (2.0 mmol) of polytetramethylene glycol (PTMG; average molecular weight, 1,000) and 0.48 ml (6.0 mmol) of pyridine were stirred in 15 ml of N,N-dimethylacetamide (DMAc) at 17°C, and 609 mg (3.0 mmol) of terephthalic acid dichloride was added to carry out 24 hours of reaction. Gelatinized substance and unreacted cellobiose were removed by filtration, and the resulting reaction solution was poured into water to effect precipitation. This was washed with water, dried under a reduced pressure, dissolved again in DMAc, re-precipitated with water, washed with water and then dried to obtain 2.06 g (yield, 75%) of the product (to be referred to as "copolymer 1" hereinafter). Its molecular weight was found to be 14,000.

### Reference Example 2

684 mg (2.0 mmol) of cellobiose, 2.0 g (2.0 mmol) of polytetramethylene glycol (PTMG; average molecular weight, 1,000) and 0.64 ml (8.0 mmol) of pyridine were stirred in 20 ml of N,N-dimethylacetamide (DMAc) at 17°C, and 812 mg (4.0 mmol) of terephthalic acid dichloride was added to carry out 24 hours of reaction. Gelatinized matter and un-reacted cellobiose were removed by filtration, and the resulting reaction solution was poured into water to effect precipitation. This was washed with water, dried under a reduced pressure, dissolved again in DMAc, re-precipitated with water, washed with water and then dried to obtain 2.61 g (yield, 81%) of the product (to be referred to as "copolymer 2" hereinafter). Its molecular weight was found to be 20,000.

### Reference Example 3

Pigment-containing tablets were produced by mixing 2% of Blue dextran 2000, 2% of dextran, 2% of Prussian blue 8GS, 2% of aniline blue, 2% of methylene blue, 3% of magnesium stearate and 87% of Avicel PH-102 (trade name, manufactured by Asahi Kasei Kogyo Kabushiki Kaisya), and treating the resulting mixture with an HU-T type tablet making machine (manufactured by Hata Iron Works) under conditions of 4 mm in the die/punch system, 30 mg in the suction quantity, 8.8 rpm in the revolving speed and 900 kg in the tablet making pressure.

### Example 1

The copolymer obtained in Reference Example 1 or 2 was added to various solvents shown in Table 1 to final concentrations of 1 to 30% (% by weight) and stirred for 24 hours or more using a stirrer. Thereafter, the solubility in each solvent was evaluated based on the following criteria.

### Criteria for solubility

- A:: Completely dissolved, yielding transparent solution.
- B:: Almost dissolved, yielding slightly turbid solution, or uniformly dispersed with no precipitation of copolymer.
- C:: Insoluble or swollen copolymer.

In addition, each of the aforementioned solutions obtained by adding the copolymer to various solvents was injected into a film-forming apparatus and dried for 48 hours at 25°C and then for 5 hours at 50°C in an oven, and properties of the thus formed films were evaluated based on the following criteria.

### Criteria for film properties

- ⓞ:: Transparent film with smooth surface
- ○:: Film with slightly irregular surface
- △:: Film with irregular or slightly patterned surface
- X:: No film formation
The results are shown in Table 1.

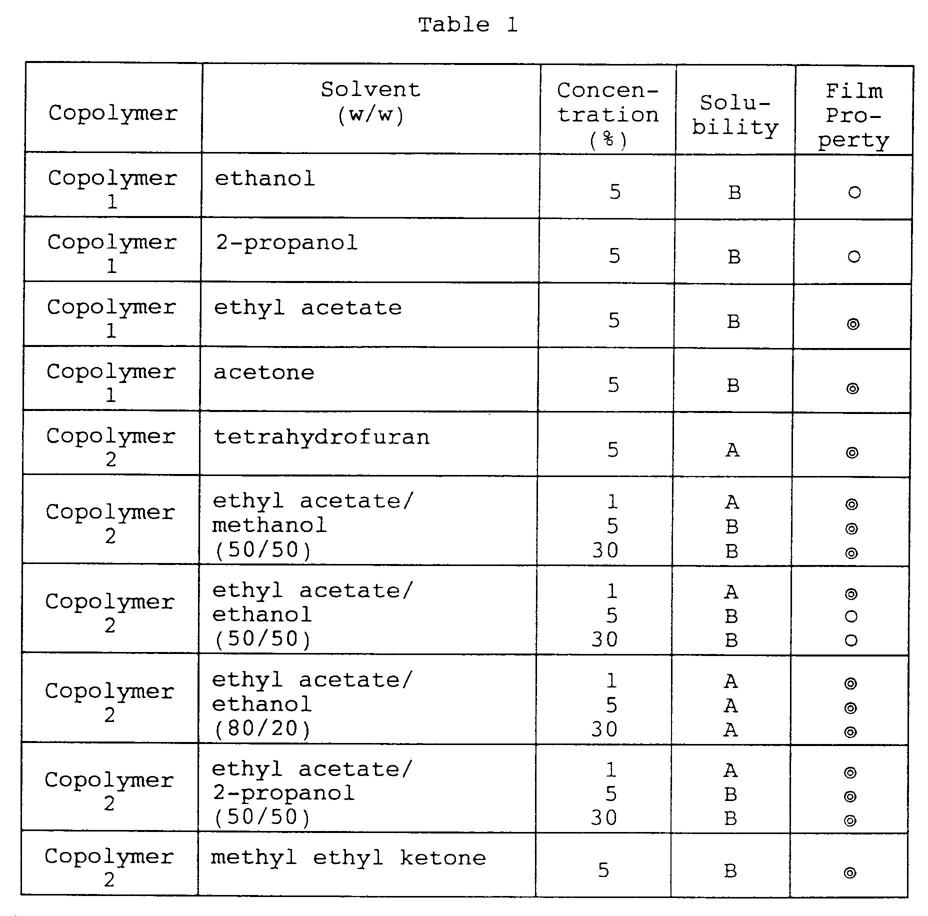

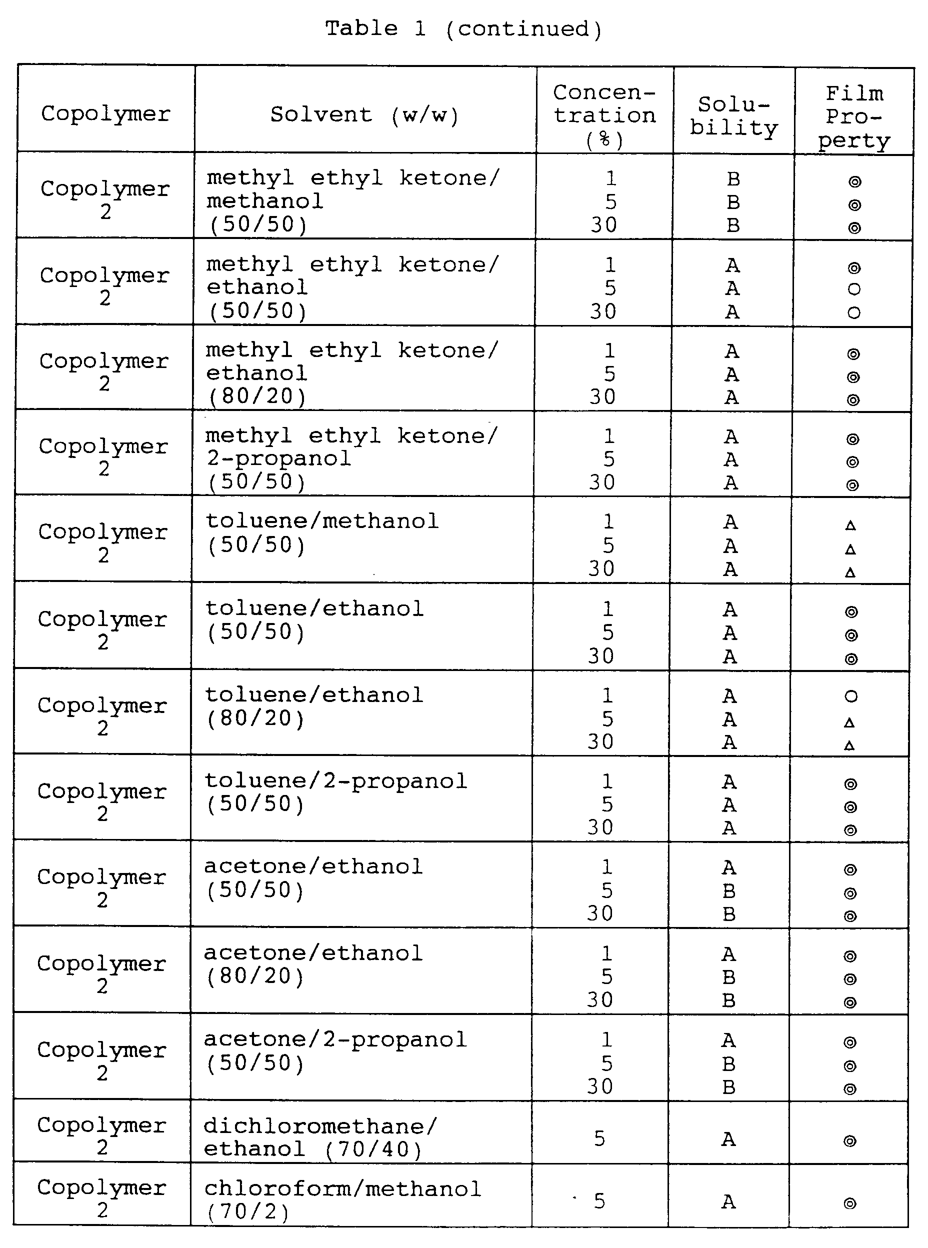

As is evident from Table 1, the copolymer of the composition of the present invention shows excellent solubility or dispersion ability in solvents. It is evident also that coating compositions in which the copolymer is dissolved or dispersed in these solvents have excellent film-forming ability.

### Comparative Example 1

The copolymer 2 obtained in Reference Example 2 was added to various solvents shown in Table 2 to respective final concentrations shown in Table 2 and stirred for 24 hours or more using a stirrer. Thereafter, its solubility in each solvent and properties of the resulting films were evaluated in the same manner as described in Example 1.

**Table 2**

| Copolymer | Solvent (w/w) | Concentration (%) | Solubility | Film Property |
|---|---|---|---|---|
| Copolymer 2 | dimethyl sulfoxide | 5 | A | ⓞ |
| Copolymer 2 | N,N-dimethylformamide | 5 | A | ⓞ |
| Copolymer 2 | N,N-dimethylformamide | 5 | A | ⓞ |

### Example 2

The copolymer 1 obtained in Reference Example 1 was dissolved in a 1:1 (volume ratio) mixed solution of dichloromethane and ethyl alcohol to a final concentration of 3%. The pigment-containing tablet obtained in Reference Example 3 was soaked in the thus prepared solution and then dried, and this step was repeated until thickness of the coated film became 5 µm. The film thickness was calculated from the difference in diameter and thickness of the tablet before and after the coating.

### Example 3

The copolymer 1 obtained in Reference Example 1 was dissolved in a 1:1 (volume ratio) mixed solution of dichloromethane and ethyl alcohol to a final concentration of 5%. The pigment-containing tablet obtained in Reference Example 3 was soaked in the thus prepared solution and then dried, and this step was repeated until thickness of the coated film became 10 µm. The film thickness was calculated from the difference in diameter and thickness of the tablet before and after the coating.

### Example 4

The copolymer 1 obtained in Reference Example 1 was dissolved in a 1:1 (volume ratio) mixed solution of dichloromethane and ethyl alcohol to a final concentration of 5%. The pigment-containing tablet obtained in Reference Example 3 was soaked in the thus prepared solution and then dried, and this step was repeated until thickness of the coated film became 20 µm. The film thickness was calculated from the difference in diameter and thickness of the tablet before and after the coating.

### Example 5

The copolymer 1 obtained in Reference Example 1 was dissolved in a 1:1 (volume ratio) mixed solution of dichloromethane and ethyl alcohol to a final concentration of 10%. The pigment-containing tablet obtained in Reference Example 3 was soaked in the thus prepared solution and then dried, and this step was repeated until thickness of the coated film became 80 µm. The film thickness was calculated from the difference in diameter and thickness of the tablet before and after the coating.

### Example 6

The copolymer 1 obtained in Reference Example 1 was dissolved in a 1:1 (volume ratio) mixed solution of dichloromethane and ethyl alcohol to a final concentration of 10%. The pigment-containing tablet obtained in Reference Example 3 was soaked in the thus prepared solution and then dried, and this step was repeated until thickness of the coated film became 150 µm. The film thickness was calculated from the difference in diameter and thickness of the tablet before and after the coating.

### Example 7

The copolymer 1 obtained in Reference Example 1 was dissolved in a 1:1 (volume ratio) mixed solution of dichloromethane and ethyl alcohol to a final concentration of 10%. The pigment-containing tablet obtained in Reference Example 3 was soaked in the thus prepared solution and then dried, and this step was repeated until thickness of the coated film became 200 µm. The film thickness was calculated from the difference in diameter and thickness of the tablet before and after the coating.

### Example 8

The copolymer 1 obtained in Reference Example 1 was dissolved in a 1:1 (volume ratio) mixed solution of dichloromethane and ethyl alcohol to a final concentration of 10%. The pigment-containing tablet obtained in Reference Example 3 was soaked in the thus prepared solution and then dried, and this step was repeated until thickness of the coated film became 300 µm. The film thickness was calculated from the difference in diameter and thickness of the tablet before and after the coating.

### Comparative Example 2

The copolymer 1 obtained in Reference Example 1 was dissolved in a 1:1 (volume ratio) mixed solution of dichloromethane and ethyl alcohol to a final concentration of 2%. The pigment-containing tablet obtained in Reference Example 3 was soaked in the thus prepared solution and then dried, and this step was repeated until thickness of the coated film became 3 µm. The film thickness was calculated from the difference in diameter and thickness of the tablet before and after the coating.

### Comparative Example 3

The copolymer 1 obtained in Reference Example 1 was dissolved in a 1:1 (volume ratio) mixed solution of dichloromethane and ethyl alcohol to a final concentration of 10%. The pigment-containing tablet obtained in Reference Example 3 was soaked in the thus prepared solution and then dried, and this step was repeated until thickness of the coated film became 350 µm. The film thickness was calculated from the difference in diameter and thickness of the tablet before and after the coating.

### Test Example 1

The pharmaceutical preparations obtained in Examples 2 to 8 and Comparative Examples 2 and 3 were checked for their disintegration and dissolution properties in the living body. These tests were carried out using 10 Wistar rats of 8 weeks of age per group.

Each of the group 1 rats was subjected to ventrotomy and the connecting part of the small intestines and the large intestine was ligated. The small intestine side of the ligated part was incised, the large intestine side was returned into the abdominal area as such, and the incised portion of the small intestines and the part of the abdominal section were sutured in such a manner that the intestinal tract remained opened outside the body. Next, the left side abdominal potion was opened by ventrotomy to incise the uppermost part of the duodenum (just below gastric pylorus), and the tablets obtained in Examples 2 to 8 and Comparative Examples 2 and 3, ten tablets for each example, were administered into the incised duodenum with a dose of 1 tablet per 1 rat. The incised duodenum portion was sutured and adhered with a surgical adhesive and the abdominal section was sutured. The thus treated rats were reared under a clean environment, and the contents exuded from the small intestinal portion remained opened were observed to examine the presence and shape of the tablets and dissolution of pigments.
The results are shown in Table 3.

**Table 3**

| (Group 1) | | | |
|---|---|---|---|
| Tablet Tested | Thickness of film | Contents colored (animals) Tablets disintegrated (animals) | Contents uncolored (animals) Shape of tablets unchanged (animals) |
| Comparative Example 2 | 3 ± 1 | 9 | 1 |
| | | 6 | 4 |
| Example 2 | 5 ± 2 | 3 | 7 |
| | | 1 | 9 |
| Example 3 | 10 ± 3 | 1 | 9 |
| | | 1 | 9 |
| Example 4 | 20 ± 3 | 0 | 10 |
| | | 0 | 10 |
| Example 5 | 80 ± 3 | 0 | 10 |
| | | 0 | 10 |
| Example 6 | 150 ± 5 | 0 | 10 |
| | | 0 | 10 |
| Example 7 | 200 ± 5 | 0 | 10 |
| | | 0 | 10 |
| Example 8 | 300 ± 7 | 0 | 10 |
| | | 0 | 10 |
| Comparative Example 3 | 350 ± 9 | 0 | 10 |
| | | 0 | 10 |

### Test Example 2

In the same manner as described in Test Example 1, the connecting portion of the small intestines and the large intestine of each of the group 2 rats was incised by the same procedure of the group 1 and then sutured and restored, and the tablets obtained in Examples 2 to 8 and Comparative Examples 2 and 3, ten tablets for each example, were administered into the duodenum from the uppermost part of the duodenum with a dose of 1 tablet per 1 rat. After sewing up the incised portion and the abdominal section, the thus treated rats were reared under a clean environment, and the fecal conditions (color tones and contents) were observed to examine the presence and shape of the tablets and dissolution of pigments. The results are shown in Table 4.

**Table 4**

| (Group 2) | | | |
|---|---|---|---|
| Tablet Tested | Thickness of film | Feces colored (animals) Tablets disintegrated (animals) | Feces uncolored (animals) Shape of tablets unchanged (animals) |
| Comparative Example 2 | 3 ± 1 | 10 | 0 |
| | | 10 | 0 |
| Example 2 | 5 ± 2 | 10 | 0 |
| | | 10 | 0 |
| Example 3 | 10 ± 3 | 10 | 0 |
| | | 10 | 0 |
| Example 4 | 20 ± 3 | 10 | 0 |
| | | 10 | 0 |
| Example 5 | 80 ± 3 | 10 | 0 |
| | | 10 | 0 |
| Example 6 | 150 ± 5 | 10 | 0 |
| | | 10 | 0 |
| Example 7 | 200 ± 5 | 9 | 1 |
| | | 9 | 1 |
| Example 8 | 300 ± 7 | 8 | 2 |
| | | 7 | 3 |
| Comparative Example 3 | 350 ± 9 | 3 | 7 |
| | | 2 | 8 |

### Test Example 3

In the same manner as described in Test Example 1, each of the group 3 rats was subjected to ventrotomy, the connecting portion of the small intestines and the large intestine was incised and then the tablets obtained in Examples 2 to 8 and Comparative Examples 2 and 3, ten tablets for each example, were administered into the large intestine with a dose of 1 tablet per 1 rat. After sewing up the incised portion and the abdominal section, the thus treated rats were reared under a clean environment, and the fecal conditions (color tones and contents) were observed to examine the presence and shape of the tablets and dissolution of pigments.

The results are shown in Table 5.

**Table 5**

| (Group 3) | | | |
|---|---|---|---|
| Tablet Tested | Thickness of film | Feces colored (animals) Tablets disintegrated (animals) | Feces uncolored (animals) Shape of tablets unchanged (animals) |
| Comparative Example 2 | 3 ± 1 | 10 | 0 |
| | | 10 | 0 |
| Example 2 | 5 ± 2 | 10 | 0 |
| | | 10 | 0 |
| Example 3 | 10 ± 3 | 10 | 0 |
| | | 10 | 0 |
| Example 4 | 20 ± 3 | 10 | 0 |
| | | 10 | 0 |
| Example 5 | 80 ± 3 | 10 | 0 |
| | | 10 | 0 |
| Example 6 | 150 ± 5 | 10 | 0 |
| | | 10 | 0 |
| Example 7 | 200 ± 5 | 8 | 2 |
| | | 8 | 2 |
| Example 8 | 300 ± 7 | 8 | 2 |
| | | 7 | 3 |
| Comparative Example 3 | 350 ± 9 | 2 | 8 |
| | | 2 | 8 |

As is evident from Tables 3, 4 and 5, the drug release-controlling preparation of the present invention does not disintegrate in the small intestines and the drug release is also inhibited. In the large intestine, on the other hand, the preparation was disintegrated and the active ingredient was released.

### Example 9

Prednisolone (5 mg) was mixed with 300 g of crystalline cellulose (trade name, Avicel PH-102, manufactured by Asahi Kasei Kyogyo Kabushiki Kaisya), and the mixture was further mixed with a lubricant mixture composed of 3 g of sodium stearate and 27 g of talc to obtain a granular preparation which was then pressed to prepare tablets, each tablet having a diameter of 13.5 mm and a weight of 500 mg and containing 5 mg of prednisolone. To the thus obtained tablets was sprayed a film-forming composition prepared by dissolving the polymer of Reference Example 1 in a 1:1 (w/w) mixed solution of dichloromethane and ethyl alcohol to a concentration of 10%, followed by drying to obtain an oral administration preparation having a coat layer of about 50 µm in thickness.

### Example 10

Release of prednisolone from the oral administration preparation obtained in Example 9 was evaluated in artificial intestinal fluids 1 (0.1 N hydrochloric acid (pH 1.2)) and 2 (phosphate buffer (pH 7.2)) and rat intestinal contents. The amount of eluted prednisolone was measured after a predetermined period of time by HPLC.

### a) Release in artificial intestinal fluids 1 and 2

A 500 mg portion (containing 5 mg of prednisolone) of the oral administration preparation obtained in Example 9 was put into 900 ml of each dissolution test solution to carry out the test at 37°C and at an agitation speed of 100 rpm. The released amounts after 3 hours and 6 hours in both test solutions were all below the detection limit.

### b) Release in rat intestinal contents

A 500 mg portion of the oral administration preparation obtained in Example 9 was incubated at 37°C in the rat intestinal contents (50 ml) to measure released amount of prednisolone at predetermined intervals. Release of prednisolone was detected after 1 hour of the incubation and 50% or more of its release was confirmed after 4 hours.

### Example 11

A 250 mg portion of 5-ASA was mixed with 250 g of crystalline cellulose (trade name, Avicel PH-102), and the mixture was further mixed with a lubricant mixture composed of 3 g of sodium stearate and 27 g of talc to obtain a granular preparation which was then pressed to prepare tablets, each tablet having a diameter of 13.5 mm and a weight of 500 mg and containing 250 mg of 5-ASA. To the thus obtained tablets was sprayed a film-forming composition prepared by dissolving the polymer of Reference Example 1 in a 1:1 (w/w) mixed solution of dichloromethane and ethyl alcohol to a concentration of 10%, followed by drying to obtain an oral administration preparation having a coat layer of about 50 µm in thickness.

### Example 12

Release of 5-ASA from the oral administration preparation obtained in Example 11 was evaluated in artificial intestinal fluids 1 and 2 and rat intestinal contents. The amount of eluted 5-ASA was measured after a predetermined period of time by a spectrometry.

### a) Release in artificial intestinal fluids 1 and 2

A 500 mg portion (containing 250 mg of 5-ASA) of the oral administration preparation obtained in Example 11 was put into 900 ml of each dissolution test solution to carry out the test at 37°C and at an agitation speed of 100 rpm. The released amounts after 3 hours and 6 hours in both test solutions were all below the detection limit.

### b) Release in rat intestinal contents

A 500 mg portion of the oral administration preparation obtained in Example 11 was incubated at 37°C in the rat intestinal contents (50 ml) to measure released amount of 5-ASA at predetermined intervals. Release of 5-ASA was detected after 1 hour of the incubation and 50% or more of its release was confirmed after 4 hours.

### INDUSTRIAL APPLICABILITY

By the use of the drug release-controlling preparation of the present invention in which a uniform coat layer having controlled thickness is formed making use of a base material containing a large intestine-specific degradable copolymer, drugs can be delivered with a high concentration into the large intestine selectively and accurately without causing their degradation and absorption in other organs than the large intestine, even by oral administration. Accordingly, the dose of drugs can be decreased, their side effects can be reduced and the dosage form design with improved extent of bioavailability is possible. Also, according to the coating composition of the present invention which contains a copolymer that shows large intestine-specific degradation, kinds and mixing ratio of solvents and concentration of the copolymer can be controlled as occasion demands and its film-forming ability is excellent, so that high concentration coating, control of coat film thickness and uniform coating of drugs by simple handling are possible by using this composition. In addition, according to the present invention, an oral administration preparation is provided which can release drugs in a large intestine-selective manner accurately and effectively and is therefore markedly useful for the treatment of inflammatory intestinal diseases, in which a composition containing an inflammatory intestinal disease-treating agent is coated with a large intestine-degradable polymer or a film-forming composition containing the polymer in a specific coat thickness.

## Claims

1. A drug release-controlling preparation which comprises a copolymer comprising at least one structural unit represented by the general formula (I): 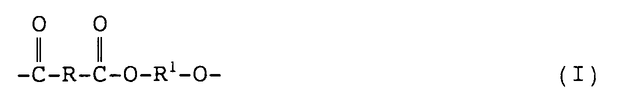 (wherein R represents a divalent organic group and R¹ represents a saccharide residue which is degraded or assimilated in the large intestine) and at least one structural unit represented by the general formula (II): 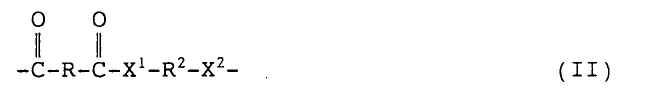 (wherein R represents a divalent organic group, X¹ and X² independently represent an oxygen atom, an imino group or a sulfur atom, and R² represents a saturated or unsaturated hydrocarbon group, a polyalkylene group, a polyalkylene glycol group, a polyarylene oxide group, a polyester group or a polyamide group), and a composition which contains an active ingredient, wherein said composition is coated with said copolymer.

2. A drug release-controlling preparation which comprises a mixture of a copolymer comprising at least one structural unit represented by the general formula (I): 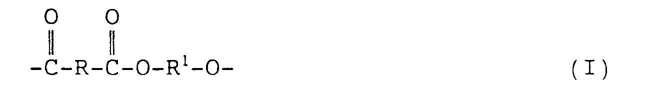 (wherein R represents a divalent organic group and R¹ represents a saccharide residue which is degraded or assimilated in the large intestine) and at least one structural unit represented by the general formula (II): 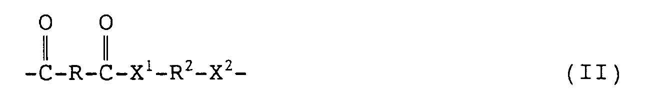 (wherein R represents a divalent organic group, X¹ and X² independently represent an oxygen atom, an imino group or a sulfur atom, and R² represents a saturated or unsaturated hydrocarbon group, a polyalkylene group, a polyalkylene glycol group, a polyarylene oxide group, a polyester group or a polyamide group) and an active ingredient.

3. The drug release-controlling preparation according to claim 1 or 2, wherein R¹ of said copolymer is an oligosaccharide residue which comprises at least two saccharide residues that are degraded or assimilated in the large intestine.

4. The drug release-controlling preparation according to claim 1 or 2, wherein R¹ of said copolymer is a saccharide residue which is hydrolyzed by β-glucosidase, β-galactosidase or β-glucuronidase.

5. The drug release-controlling preparation according to claim 1 or 2, wherein R¹ of said copolymer is a galacto-oligosaccharide residue, a fructo-oligosaccharide residue or a soybean oligosaccharide residue.

6. The drug release-controlling preparation according to claim 1 or 2, wherein R¹ of said copolymer is cellobiose.

7. The drug release-controlling preparation according to claim 1 or 2, wherein the structural unit (I) of said copolymer is a structural unit obtained by ester-bonding a dibasic acid represented by HOOC-R-COOH and a saccharide represented by HO-R¹-OH, and the structural unit (II) of said copolymer is a structural unit obtained by ester-bonding a dibasic acid represented by HOOC-R-COOH and a polyalkylene glycol represented by HO-R²-OH.

8. The drug release-controlling preparation according to claim 7, wherein the dibasic acid represented by HOOC-R-COOH constituting the structural unit (I) of said copolymer is terephthalic acid or isophthalic acid and the saccharide represented by HO-R¹-OH constituting the same is cellobiose, and the dibasic acid represented by HOOC-R-COOH constituting the structural unit (II) of said copolymer is terephthalic acid or isophthalic acid and the polyalkylene glycol represented by HO-R²-OH constituting the same is polytetramethylene glycol or polypropylene glycol.

9. The drug release-controlling preparation according to claim 1 or 2, wherein said active ingredient is a drug for the treatment of inflammatory intestinal diseases.

10. The drug release-controlling preparation according to claim 9, wherein said drug for the treatment of inflammatory intestinal diseases is a steroid drug or a pharmaceutically acceptable salt thereof.

11. The drug release-controlling preparation according to claim 10, wherein said drug for the treatment of inflammatory intestinal diseases is prednisolone.

12. The drug release-controlling preparation according to claim 9, wherein said drug for the treatment of inflammatory intestinal diseases is aminosalicylic acid or an aminosalicylic acid derivative, or a pharmaceutically acceptable salt thereof.

13. The drug release-controlling preparation according to claim 1 or 2, wherein its dosage form is tablets, capsules or granules.

14. The drug release-controlling preparation according to claim 1 or 2 wherein the coat film of said copolymer has a thickness of approximately from 5 to 300 µm.

15. A composition which comprises a solvent and a copolymer comprising at least one structural unit represented by the general formula (I): 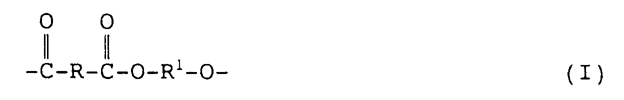 (wherein R represents a divalent organic group and R¹ represents a saccharide residue which is degraded or assimilated in the large intestine) and at least one structural unit represented by the general formula (II): 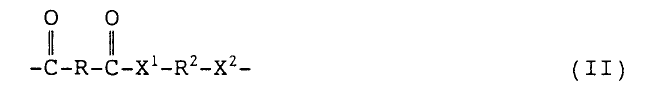 (wherein R represents a divalent organic group, X¹ and X² independently represent an oxygen atom, an imino group or a sulfur atom, and R² represents a saturated or unsaturated hydrocarbon group, a polyalkylene group, a polyalkylene glycol group, a polyarylene oxide group, a polyester group or a polyamide group), said copolymer being dissolved or dispersed in said solvent.

16. The composition according to claim 15, wherein R¹ of said copolymer is an oligosaccharide residue which comprises at least two saccharide residues that are degraded or assimilated in the large intestine.

17. The composition according to claim 15, wherein R¹ of said copolymer is a saccharide residue which is hydrolyzed by β-glucosidase, β-galactosidase or β-glucuronidase.

18. The composition according to claim 15, wherein R¹ of said copolymer is a galacto-oligosaccharide residue, a fructo-oligosaccharide residue or a soybean oligosaccharide residue.

19. The composition according to claim 15, wherein R¹ of said copolymer is cellobiose.

20. The composition according to claim 15, wherein the structural unit (I) of said copolymer is a structural unit obtained by ester-bonding a dibasic acid represented by HOOC-R-COOH and a saccharide represented by HO-R¹-OH, and the structural unit (II) of said copolymer is a structural unit obtained by ester-bonding a dibasic acid represented by HOOC-R-COOH and a polyalkylene glycol represented by HO-R²-OH.

21. The composition according to claim 15, wherein the dibasic acid represented by HOOC-R-COOH constituting the structural unit (I) of said copolymer is terephthalic acid or isophthalic acid and the saccharide represented by HO-R¹-OH constituting the same is cellobiose, and the dibasic acid represented by HOOC-R-COOH constituting the structural unit (II) of said copolymer is terephthalic acid or isophthalic acid and the polyalkylene glycol represented by HO-R²-OH constituting the same is polytetramethylene glycol or polypropylene glycol.

22. The composition according to claim 15, wherein said copolymer is dissolved or dispersed in a solvent in a concentration of from 0.1 to 50% (% by weight) based on the total weight of said composition.

23. The composition according to claim 15, wherein said solvent comprises at least one solvent selected from the group consisting of water, alcohols having 1 to 5 carbon atoms, aromatic hydrocarbons, esters, ketones, ethers, halides and glycols.

24. The composition according to claim 23, wherein said solvent is tetrahydrofuran.

25. The composition according to claim 23, wherein said solvent is a mixed solvent of methanol, ethanol or 2-propanol with toluene.

26. The composition according to claim 23, wherein said solvent is a mixed solvent of methanol, ethanol or 2-propanol with ethyl acetate.

27. The composition according to claim 23, wherein said solvent is a mixed solvent of methanol, ethanol or 2-propanol with methyl ethyl ketone.

28. The composition according to claim 23, wherein said solvent is a mixed solvent of dichloromethane or chloroform with methanol.

29. The composition according to claim 23, wherein said solvent is a mixed solvent of dichloromethane or chloroform with ethanol.

30. The composition according to claim 23, wherein said solvent is water.

31. The composition according to claim 23, wherein said solvent is selected from alcohols having 1 to 5 carbon atoms.

32. The composition according to claim 23, wherein said solvent is methyl ethyl ketone.

33. The composition according to claim 23, wherein said solvent is a mixed solvent of methanol, ethanol or 2-propanol with acetone.
